# EUROPEAN PATENT SPECIFICATION

(11) **EP 2 946 809 B1**
(45) Date of publication and mention of the grant of the patent: **14.08.2019**
(21) Application number: 15152686.0
(22) Date of filing: 27.01.2015
(51) Int. Cl.: A61N 5/10, H05H 3/06, G01R 19/00

(54) **Neutron capture therapy apparatus and nuclear transformation apparatus**
Neutroneneinfangtherapievorrichtung und Nukleartransformationsvorrichtung
Appareil de thérapie par capture de neutrons et appareil de transformation nucléaire

(30) Priority: 20.05.2014 JP 2014104273
(43) Date of publication of application: 25.11.2015
(73) Proprietor: SUMITOMO HEAVY INDUSTRIES, LTD., Tokyo 141-6025 (JP)
(72) Inventor: Mitsumoto, Toshinori, Tokyo 188-8585 (JP); Kikuchi, Yuuji, Ehime 792-8588 (JP)
(74) Representative: Schmidbauer, Andreas Konrad

(56) References cited:
- EP-A1- 1 895 819
- EP-A1- 2 110 844
- EP-A1- 2 600 356
- EP-A2- 1 329 938
- WO-A1-92/12415
- DE-A1- 2 363 490
- US-A- 3 014 132

## Description

### BACKGROUND OF THE INVENTION

### Field of the Invention

The present invention relates to a neutron capture therapy apparatus which irradiates an irradiation object with a neutron beam, and a nuclear transformation apparatus.

### Description of Related Art

In the related art, an apparatus disclosed in WO 2012/014671 A1 is known as an apparatus used in neutron capture therapy by which cancer cells are exterminated by being irradiated with a neutron beam. The neutron capture therapy apparatus disclosed in WO 2012/014671 A1 includes an accelerator emitting a charged particle beam; a target which is irradiated with the charged particle beam to generate a neutron beam; and a beam transport path through which the charged particle beam emitted by the accelerator is transported to the target.

In the neutron capture therapy apparatus disclosed in the above-described WO 2012/014671 A1, the total amount of charged particle beams with which a target is irradiated is detected by an ammeter. However, there is a problem in that it is impossible to detect whether or not the position at which the target is irradiated with the charged particle beam is normal.

In addition, a current value of the charged particle beam used in the neutron capture therapy is extremely high compared to that of a proton beam treatment apparatus. For this reason, when applying a wire grid beam position monitor, which is used in the proton beam treatment apparatus or the like, to the neutron capture therapy apparatus, the beam position monitor is exposed to the charged particle beam with a high current value. As a result, there is a possibility that the beam position monitor will deteriorate. Accordingly, it is difficult to use such a beam position monitor in the neutron capture therapy apparatus.

DE23 63 4 90 A discloses a source of high velocity and current density neutrons for application in radiation biology, deep tissue therapy etc. The neutrons are generated by utilising nuclear reactions. The target material of the source is a gas enclosed in a pressure chamber with an inlet window for the particles stimulating the nuclear reaction. This inlet window is provided with a cooling arrangement, a second cooling arrangement being provided in the pressure chamber for carrying away the reaction heat generated in the target material. Preferably the inlet window separates the pressure chamber from a vacuum space . The pressure chamber may contain a cooling cylinder whose forward open face points in the direction of the inlet window and whose axis is located in the radiation direction of the stimulating particles. In the beam tube, an orifice arrangement having an orifice and a cooling device is provided, the orifice limiting the ion beam. The orifice arrangement acts like a Faraday cup. By means of an electric feed-through, the orifice current can be detected and measured.

EP 2 600 356 A1 discloses a neutron beam irradiation apparatus that irradiates an irradiation object with a neutron beam includes charged particle beam generating means for generating a charged particle beam, neutron beam generating means for generating the neutron beam by irradiating a target with the charged particle beam, and measuring means for measuring a dose of the charged particle beam in real time during irradiation with the neutron beam.

### SUMMARY OF THE INVENTION

An object of the present invention is to provide a neutron capture therapy apparatus which can detect whether or not a position at which a target is irradiated with a charged particle beam is normal, and a nuclear transformation apparatus.

According to an aspect of the present invention, there is provided a neutron capture therapy apparatus as set forth in claim 1.

According to another aspect of the present invention, there is provided a nuclear transformation apparatus as set forth in claim 6.

In the neutron capture therapy apparatus and the nuclear transformation apparatus according to the present invention, the current detection portion which detects the current value of the charged particle beam is provided inside the beam transport path along the inner wall of the beam transport path in a state of being insulated from the inner wall, and therefore, it is possible to detect a current value of a charged particle beam in a low current region which is close to the inner wall. Accordingly, the current detection portion is hardly exposed to a charged particle beam in a high current region, thereby suppressing deterioration. Accordingly, it is possible to detect whether or not a position at which the target is irradiated with the charged particle beam is normal based on the current value detected by such a current detection portion.

In addition, in the neutron capture therapy apparatus according to the present invention, the current detection portion may be formed in an annular shape. In this case, it is possible to detect a minute current value of the charged particle beam in the low current region which encloses the charged particle beam in the high current region.

In addition, in the neutron capture therapy apparatus according to the present invention, a plurality of the current detection portions may be provided along the inner wall. In this case it is possible to detect the current value of the charged particle beam using the plurality of current detection portions . Accordingly, it can be seen which current detection portion is brought into contact with a charged particle beam in an abnormal state based on the current value detected by each of the current detection portions. Accordingly, it is possible to obtain information on which position of a current detection portion is irradiated with the charged particle beam in the abnormal state.

In addition, in the neutron capture therapy apparatus according to the present invention, the current detection portion may be provided so as to protrude from the inner wall side of the beam transport path to the inside of the beam transport path. In this case, the current detection portion along the inner wall of the beam transport path is provided so as to protrude from the inner wall side to the inside of the beam transport path. Therefore, it is possible to favorably detect the minute current value of the charged particle beam in the low current region which encloses the charged particle beam in the high current region.

In addition, the neutron capture therapy apparatus according to the present invention may further include a control portion which controls emission of the charged particle beam based on the size of the current value detected by the current detection portion. In this case, it is possible to control the irradiation of the target with the charged particle beam using the control portion by assuming that the position at which a target is irradiated with a charged particle beam is abnormal when a current value less than or equal to a lower limit value which is smaller than a reference current value, or a current value greater than or equal to an upper limit value which is greater than the reference current value is detected, by setting a current value detected by the current detection portion when the position at which the target is irradiated with the charged particle beam is normal, as the reference current value.

According to the present invention, it is possible to provide the neutron capture therapy apparatus which can detect whether or not the target is irradiated with a charged particle beam at a normal position, and the nuclear transformation apparatus.

### BRIEF DESCRIPTION OF THE DRAWINGS

Fig. 1 is a view showing a configuration of a neutron capture therapy apparatus according to a first embodiment.
Fig. 2 is a perspective view showing the outline of a neutron beam generating portion in the neutron capture therapy apparatus of Fig. 1.
Fig. 3 is a side cross-sectional view showing a schematic configuration of a current detection portion shown in Fig. 2.
Fig. 4 is a transverse cross-sectional view taken along line IV-IV shown in Fig. 3.
Fig. 5 is a side cross-sectional view showing an example of a mounting structure of the current detection portion shown in Fig. 2.
Figs. 6A and 6B are schematic views showing abnormal states of positions at which targets are irradiated with charged particle beams.
Fig. 7 is a transverse cross-sectional view corresponding to the current detection portion in Fig. 4 which is included in a neutron capture therapy apparatus according to a second embodiment.

### DETAILED DESCRIPTION OF THE INVENTION

Hereinafter, embodiments of a neutron capture therapy apparatus according to the present invention will be described while referring to the accompanying drawings. In the following description, identical elements or corresponding elements are given the same reference numerals and the repeated description thereof will be omitted.

### First Embodiment

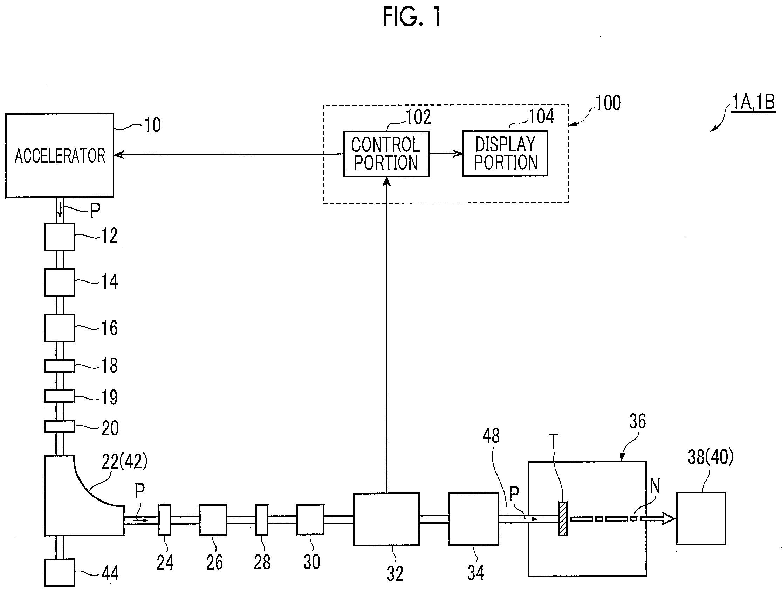

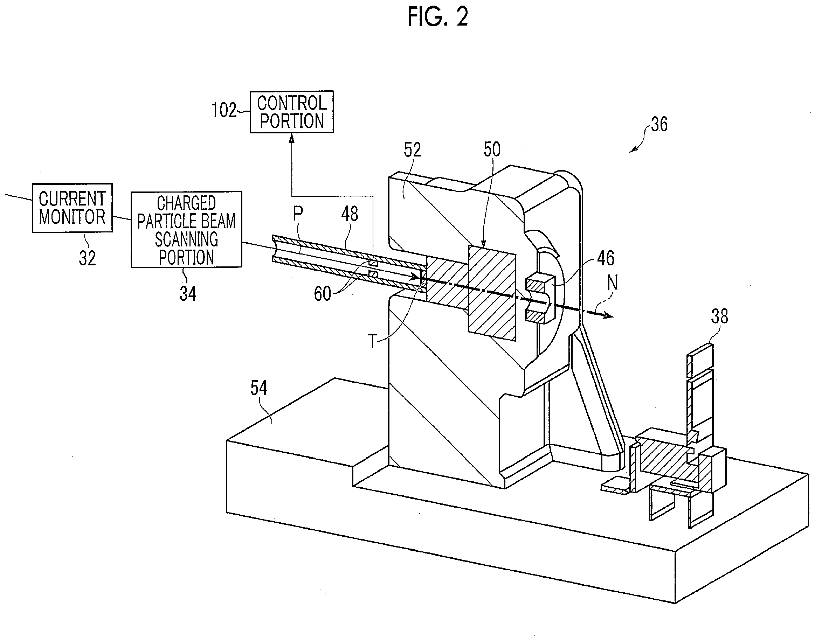

First, the outline of a neutron capture therapy apparatus according to a first embodiment will be described using Figs. 1 and 2. The neutron capture therapy apparatus according to the present embodiment is, for example, a neutron capture therapy apparatus for performing cancer treatment through boron neutron capture therapy. As shown in Figs. 1 and 2, a neutron capture therapy apparatus 1A irradiates an irradiation object 40 such as a patient, to which boron (¹⁰B) is administered, with a neutron beam N.

The neutron capture therapy apparatus 1A includes an accelerator 10. The accelerator 10 is an accelerator which generates a proton beam as a charged particle beam P by accelerating charged particles such as protons. In the present embodiment, a cyclotron is employed as the accelerator 10. The accelerator 10 has the ability to generate a charged particle beam P with, for example, a beam radius of 40 mm and 60 kW (= 30 MeV x 2 mA) . Other accelerators such as a synchrotron, a synchrocyclotron, or a linear accelerator may be used as the accelerator 10, instead of the cyclotron.

The charged particle beam P emitted from the accelerator 10 sequentially passes through a horizontal type steering unit 12, a four-direction slit 14, a horizontal/vertical type steering unit 16, quadrupole electromagnets 18, 19, and 20, a 90-degree deflecting electromagnet 22, a quadrupole electromagnet 24, a horizontal/vertical type steering unit 26, a quadrupole electromagnet 28, a four-direction slit 30, a current monitor 32, and a charged particle beam scanning portion 34, and is led to a neutron beam generating portion 36. A target T in the neutron beam generating portion 36 is irradiated with the charged particle beam P, and as a result, a neutron beam N is generated. The irradiation object 40 on a treatment table 38 is irradiated with the neutron beam N.

The horizontal type steering unit 12, and horizontal/vertical type steering units 16 and 26 suppress, for example, scattering of the charged particle beam P using an electromagnet. Similarly, the quadrupole electromagnets 18, 19, 20, 24, and 28 perform, for example, beam axis adjustment of the charged particle beam P using the electromagnets. Four-direction slits 14 and 30 perform beam shaping of the charged particle beam P by cutting the beam at an end thereof.

The 90-degree deflecting electromagnet 22 deflects the traveling direction of the charged particle beam P by 90 degrees . The 90-degree deflecting electromagnet 22 is provided with a switching portion 42, and therefore, it is possible to lead the charged particle beam P to a beam dump 44 by making the charged particle beam stray from a regular track using the switching portion 42. The beam dump 44 checks the output of the charged particle beam P before treatment or the like.

The current monitor 32 measures a current value (that is, electrical charge and irradiation dose rate) of a charged particle beam P with which a target T is irradiated in real time. A nondestructive DC current transformer (DCCT) which can measure a current without affecting the charged particle beam P is used in the current monitor 32. A controller 100 is connected to the current monitor 32. The "dose rate" means a dose per unit of time (the same applies to the following).

The controller 100 includes a control portion 102 and a display portion 104. The control portion 102 obtains an irradiation dose of a neutron beam N from a current value of a charged particle beam P measured by the current monitor 32 and controls emission of the neutron beam N based on the irradiation dose. For example, the control portion 102 consists of a CPU, a ROM, a RAM, and the like. The display portion 104 displays the irradiation dose of the neutron beam N obtained by the control portion 102, and for example, a display or a monitor is used as the display portion.

The charged particle beam scanning portion 34 scans a charged particle beam P and controls irradiation of a target T with the charged particle beam P. The charged particle beam scanning portion 34 referred herein controls, for example, the position at which the target T is irradiated with the charged particle beam P, the beam diameter of the charged particle beam P, or the like. The irradiation region of the charged particle beam P on the target T is enlarged through a wobbling operation of the charged particle beam P by the charged particle beam scanning portion 34, or through enlargement of the beam diameter of the charged particle beam P by the charged particle beam scanning portion 34. The wobbling operation refers to an operation in which a beam axis of the charged particle beam P with a constant beam diameter is periodically moved, and the area of the target T irradiated with the charged particle beam P is enlarged due to the periodic movement.

As shown in Fig. 2, the neutron beam generating portion 36 generates a neutron beam N by irradiating a target T with a charged particle beam P, and emits the neutron beam N through a collimator 46. The neutron beam generating portion 36 includes the target T which is disposed in a downstream end of the beam transport path 48 through which the charged particle beam P is transported; a moderator material 50 which reduces the neutron beam N generated in the target T; and a shielding body 52 which is provided so as to cover the target and the moderator material.

The target T generates a neutron beam N by being irradiated with a charged particle beam P. The target T referred herein is formed of, for example, beryllium (Be), and is formed into a disk shape with a diameter of 160 mm. The moderator material 50 reduces the speed of the energy of the neutron beam N and is set to have, for example, a stacked structure formed of a plurality of different materials. The shielding body 52 shields a generated neutron beam N, a gamma ray which is generated due to the generation of the neutron beam N, and the like so as not to be discharged to the outside, and is attached to a floor 54. The target T is not limited to be in a solid state (tabular shape), and may be in a liquid state.

As shown in Fig. 2, in the neutron capture therapy apparatus 1A according to the present embodiment, a current detection portion 60 which detects a current value of a charged particle beam P is included inside the beam transport path 48. The current detection portion 60 is disposed at a position between the charged particle beam scanning portion 34 and the neutron beam generating portion 36 on the inside of the beam transport path 48. An oscilloscope is connected to the current detection portion 60 through, for example, wiring. The oscilloscope displays a waveform corresponding to the current value detected by the current detection portion 60. The current value detected by the current detection portion 60 is observed based on the waveform of the oscilloscope.

The current detection portion 60 is connected to the above-described control portion 102. The control portion 102 controls emission of a charged particle beam P based on the current value of the charged particle beam P detected by the current detection portion 60. A specific control method using the control portion 102 will be described later.

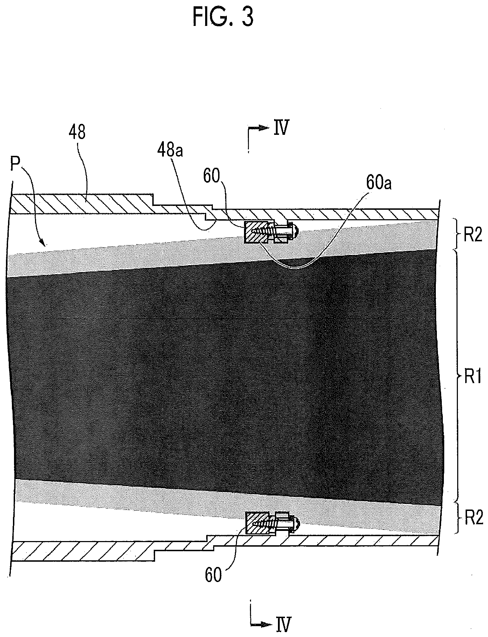

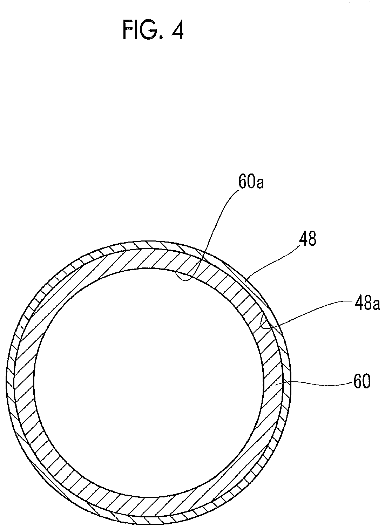

Next, a configuration of the current detection portion 60 will be described in detail with reference to Figs. 3 and 4. Fig. 3 is a side cross-sectional view showing a schematic configuration of the current detection portion 60 shown in Fig. 2. Fig. 4 is a transverse cross-sectional view taken along line IV-IV shown in Fig. 3. As shown in Figs. 3 and 4, the current detection portion 60 is formed in an annular shape along an inner wall 48a.

The current detection portion 60 has an outer circumferential surface and an inner circumferential surface which extend along a circumferential direction of the inner wall 48a with a substantially constant width and are bent at a substantially identical curvature to that of the inner wall 48a. The current detection portion 60 has a ring shape inside the inner wall 48a when seen from an axis direction of the beam transport path 48 (refer to Fig. 4). In the present embodiment, the inner wall 48a has a cylindrical shape, and therefore, the current detection portion 60 is formed in a true circular shape. The current detection portion 60 has an opening 60a as an opening through which a charged particle beam with a high current passes in a central region in a radial direction of the beam transport path 48.

The inner diameter of the current detection portion 60 is, for example, 14 cm to 17 cm, and the outer diameter of the current detection portion 60 is, for example, 16 cm to 19 cm. In the beam transport path 48, the current detection portion 60 is disposed at, for example, a position separated from a target T by approximately 35 cm.

The current detection portion 60 detects a current of a charged particle beam P by coming into contact with the charged particle beam P. The current detection portion 60 is formed of a material which has conductivity and is not easily melted by heat. For example, the current detection portion 60 is formed of a carbon material such as graphite, or pure copper, etc.

Here, as shown in Fig. 3, a high current region R1 which has a large current value (irradiation dose rate) and a low current region R2 which has a small current value (irradiation dose rate) are distributed in the charged particle beam P which passes through the beam transport path 48. The low current region R2 encloses the periphery of the high current region R1, and is a region which is closer to the inner wall 48a than the high current region R1 when the position at which the target T is irradiated with the charged particle beam P is in a normal state.

The high current region R1 is a region with a current value of, for example, approximately 1 mA to 1.5 mA. The low current region R2 is a region with a current value of, for example, approximately 2 µA to 3 µA. That is, the low current region R2 is a region with a current value having a size of, for example, approximately 0.2% of the high current region R1.

The current detection portion 60 is provided so as to protrude from the inner wall 48a side to the inside of the beam transport path 48. That is, the current detection portion 60 is configured such that the current detection portion 60 protrudes toward the inner circumferential side of the beam transport path 48 since the inner diameter of the current detection portion 60 is set to be smaller than at least the inner diameter of the inner wall 48a. Accordingly, the current detection portion 60 is configured so as to be disposed in a state where the annular end surface of the current detection portion is exposed to the inside of the beam transport path 48 as a detection surface of the charged particle beam P.

The current detection portion 60 is formed so as to come into contact with a charged particle beam P in the low current region R2 in a state where the position at which the target T is irradiated with the charged particle beam P is normal. The current detection portion 60 detects a minute current value of the charged particle beam P by coming into contact with the charged particle beam P in the low current region R2.

For example, when the beam diameter of the charged particle beam P is enlarged by the charged particle beam scanning portion 34 in a state where the position at which the target T is irradiated with the charged particle beam P is normal, the current detection portion 60 is formed so as to always come into contact with the charged particle beam P in the low current region R2 at any position in the circumferential direction of the current detection portion 60. In addition, when the charged particle beam P performs a wobbling operation using the charged particle beam scanning portion 34 in a state where the position at which the target T is irradiated with the charged particle beam P is normal, the current detection portion 60 is formed so as to come into contact with the charged particle beam P in the low current region R2 at any position in the circumferential direction of the current detection portion 60.

The current detection portion 60 is formed so as not to come into contact with the charged particle beam P in the high current region R1 in a state where the position at which the target T is irradiated with the charged particle beam P is normal. In the state where the position at which the target T is irradiated with the charged particle beam P is normal, the charged particle beam P in the high current region R1 passes through the opening 60a of the current detection portion 60.

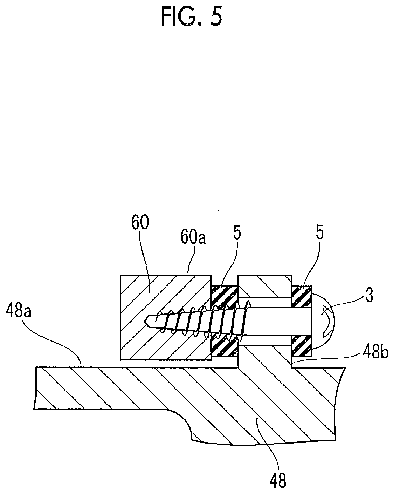

Next, an example of a mounting structure of the current detection portion 60 will be described with reference to Fig. 5. Fig. 5 is a side cross-sectional view showing an example of a mounting structure of the current detection portion 60 shown in Fig. 2. As shown in Fig. 5, the current detection portion 60 is attached to a protruding portion 48b which is formed on the inner wall 48a of the beam transport path 48 through an insulation member 5. The current detection portion 60 is attached to the protruding portion 48b using a screw 3 in a state where the insulation member 5 is interposed between the current detection portion and the protruding portion 48b. That is, the current detection portion 60 is provided in a state of being insulated from the inner wall 48a of the beam transport path 48. A plurality of fixing sites between the current detection portion 60 and the protruding portion 48b are provided at predetermined intervals when seen in a direction in which the beam transport path 48 extends. The inner wall 48a and the outer circumferential surface of the current detection portion 60 are separated from each other in order to insulate the inner wall 48a and the current detection portion 60.

Next, a control method using the control portion 102 based on the current value detected by the current detection portion 60 will be described in detail.

First, a case in which the position at which the target T is irradiated with the charged particle beam P is in a normal state will be described with reference to Fig. 3. As shown in Fig. 3, the current detection portion 60 comes into contact with the charged particle beam P in the low current region R2 in a state where the position at which the target T is irradiated with the charged particle beam P is normal. Accordingly, the current detection portion 60 detects a minute current value (for example, 2 µA) of the charged particle beam P.

The control portion 102 performs control so as to continue the irradiation of the target with the charged particle beam P as it is when the current value (hereinafter, also referred to as a reference current value) in such a normal state is detected by the current detection portion 60.

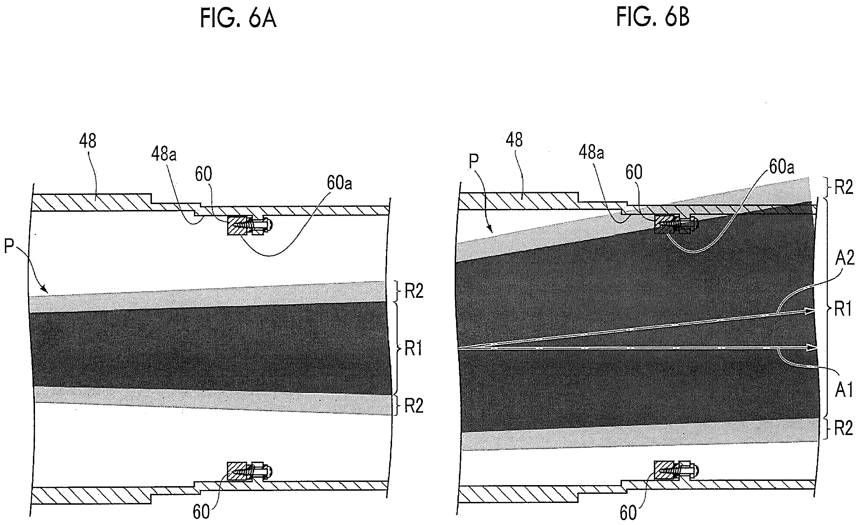

Next, a case in which the position at which the target T is irradiated with the charged particle beam P is in an abnormal state will be described with reference to Figs. 6A and 6B. Figs. 6A and 6B are schematic views showing abnormal states of positions at which targets T are irradiated with charged particle beams P.

As shown in Fig. 6A, the current detection portion 60 cannot come into contact with the charged particle beam P in the low current region R2, for example, in a case where the beam diameter of the charged particle beam P is not normally enlarged due to defects or the like in the quadrupole electromagnets 18, 19, 20, 24, and 28, or in the charged particle beam scanning portion 34, or in a case where the wobbling operation of the charged particle beam P is not normally functioning. Accordingly, the current value detected by the current detection portion 60 becomes less than or equal to the lower limit value (for example, 1 µA to 0 µA) which is smaller than the reference current value.

As shown in Fig. 6B, a part of the charged particle beam P in the high current region R1 comes into contact with the current detection portion 60, for example, in a case where the beam axis of the charged particle beam P becomes a beam axis A2 which is deviated from the beam axis A1 when the beam axis of the charged particle beam P is normal, due to defects or the like in the horizontal type steering unit 12, the horizontal/vertical type steering units 16 and 26, the quadrupole electromagnets 18, 19, 20, 24, and 28, the 90-degree deflecting electromagnet 22, or in the charged particle beam scanning portion 34. Accordingly, the current value detected by the current detection portion 60 becomes greater than or equal to the upper limit value (for example, 5 µA) which is greater than the reference current value.

When current values in the abnormal states as shown in Figs . 6A and 6B are detected by the current detection portion 60, the control portion 102 stops irradiation of a target with a charged particle beam P based on the current value. Specifically, when the current value is rarely detected as in the case of Fig. 6A, the control portion 102 stops the irradiation of the target with the charged particle beam P by assuming that the position at which the target T is irradiated with the charged particle beam P is in the abnormal state. In addition, even when the size of the detected current value becomes greater than or equal to the upper limit value which is greater than the reference current value, the control portion 102 stops the irradiation of the target with the charged particle beam P by assuming that the position at which the target T is irradiated with the charged particle beam P is in the abnormal state.

Stopping of the irradiation of the target with the charged particle beam P using the control portion 102 is performed by, for example, controlling a beam chopper (not shown) in the accelerator 10 which generates the charged particle beam P. In addition, the control portion 102 may not only stop the irradiation of the target with the charged particle beam P, but may also perform, for example, feedback control such that the position at which the target T is irradiated with the charged particle beam P becomes a normal state based on the current value in the abnormal state.

Next, the actions and effects of the neutron capture therapy apparatus 1A according to the present embodiment will be described.

In the neutron capture therapy apparatus in the related art, the total amount of charged particle beams with which a target is irradiated is detected by an ammeter. However, there is a problem in that it is impossible to detect whether or not the position at which the target is irradiated with the charged particle beam is normal.

For this reason, for example, when the charged particle beam is concentrated in the center portion of the target due to the defects or the like in the electromagnet enlarging the irradiation region, it is impossible to detect the abnormality, and therefore, there is a possibility that the thermal limit of the target is exceeded as it is. In addition, when the travelling direction of the charged particle beam is deviated due to the defects or the like in the electromagnet for beam transport, it is impossible to detect the abnormality, and therefore, there is a possibility that a vacuum tube will be melted by heat as the vacuum tube, which forms the beam transport path, is continuously irradiated with the charged particle beam.

In addition, the current value of the charged particle beam used in the neutron capture therapy is extremely high compared to that of a proton beam treatment apparatus. For this reason, when applying a wire grid beam position monitor, which is used in the proton beam treatment apparatus or the like, to the neutron capture therapy apparatus, the beam position monitor is exposed to the charged particle beam with a high current value. As a result, there is a possibility that the beam position monitor will deteriorate. Accordingly, it is difficult to use such a beam position monitor in the neutron capture therapy apparatus.

In contrast, according to the neutron capture therapy apparatus 1A of the present embodiment, the current detection portion 60 which detects a current value of a charged particle beam P is provided inside the beam transport path 48 along the inner wall 48a of the beam transport path 48 in a state of being insulated from the inner wall 48a. Therefore, it is possible to detect the current value of the charged particle beam P in the low current region R2 which is close to the inner wall 48a. Accordingly, the current detection portion 60 is hardly exposed to the charged particle beam P in the high current region R1, and therefore, deterioration of the current detection portion is suppressed. Accordingly, it is possible to detect whether or not a position at which a target T is irradiated with a charged particle beam P is normal based on the current value detected by the current detection portion 60.

In general, the current value of a charged particle beam used in a neutron capture therapy apparatus is extremely high. However, according to the neutron capture therapy apparatus 1A of the present embodiment, the current detection portion 60 is provided along the inner wall 48a of the beam transport path 48. Therefore, it is possible to detect a minute current value of the charged particle beam P in the low current region R2 which encloses the charged particle beam P in the high current region R1. Moreover, it is possible to effectively suppress deterioration of the current detection portion 60 due to exposure of the current detection portion 60 to the charged particle beam P in the high current region R1.

In the neutron capture therapy apparatus 1A, it is possible to detect the minute current value of the charged particle beam P in the low current region R2 which encloses the charged particle beam P in the high current region R1 using the current detection portion 60 with a simple structure of an annular shape.

In the neutron capture therapy apparatus 1A, the current detection portion 60 is provided so as to protrude from the inner wall 48a side of the beam transport path 48 to the inside of the beam transport path 48 along the inner wall 48a. Therefore, the current detection portion is suitable for detecting the minute current value of the charged particle beam P in the low current region R2 which encloses the charged particle beam P in the high current region R1.

Furthermore, in the neutron capture therapy apparatus 1A, the control portion 102 controls irradiation of a target with a charged particle beam P based on the size of the current value detected by the current detection portion 60. Accordingly, it is possible to control the irradiation of the target with the charged particle beam P using the control portion 102 by assuming that the position at which a target T is irradiated with a charged particle beam P is abnormal when a current value less than or equal to a lower limit value which is smaller than a reference current value, or a current value greater than or equal to an upper limit value which is greater than the reference current value is detected, by setting a current value detected by the current detection portion 60 when the position at which the target T is irradiated with the charged particle beam P is normal, as the reference current value.

In general, it is possible to obtain irradiation position information in a circumferential direction on which position along the circumferential direction of the beam transport path 48 is irradiated with the charged particle beam P during the wobbling operation. Accordingly, when an abnormal state of the irradiation position is detected due to the abnormality of the wobbling operation, it is possible to obtain information on which position along the circumferential direction of the beam transport path 48 is irradiated with the charged particle beam P when the current value shows the abnormal state, by synchronizing the current value detected by the current detection portion 60 and the irradiation position information in the circumferential direction using the wobbling operation.

### Second Embodiment

Next, a configuration of a neutron capture therapy apparatus according to a second embodiment will be described. In the description of the present embodiment, different points from those of the above-described first embodiment will be mainly described.

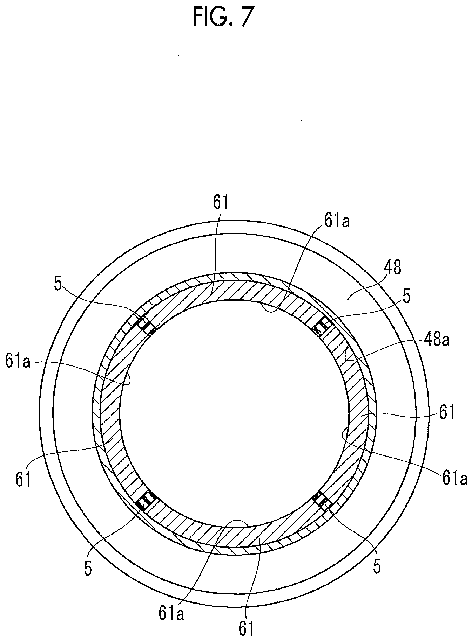

Similarly to the first embodiment, a neutron capture therapy apparatus 1B according to the present embodiment includes an accelerator 10, a target T, and a beam transport path 48 (refer to Fig. 1). The neutron capture therapy apparatus 1B is different from the neutron capture therapy apparatus 1A according to the first embodiment in terms of inclusion of a plurality of current detection portions. Hereinafter, the configuration of the current detection portion according to the present embodiment will be described with reference to Fig. 7. Fig. 7 is a transverse cross-sectional view corresponding to the current detection portion in Fig. 4 which is included in the neutron capture therapy apparatus 1B according to the second embodiment.

As shown in Fig. 7, the neutron capture therapy apparatus 1B according to the second embodiment includes, for example, four current detection portions 61 along an inner wall 48a. The spaces between the current detection portions 61 are insulated by insulating members 5. Each of the current detection portions 61 detects a current value of a charged particle beam P in a low current region R2 which is brought into contact therewith.

Similarly to the above-described first embodiment, also in the neutron capture therapy apparatus 1B according to the present embodiment, it is possible to detect whether or not a position at which a target T is irradiated with a charged particle beam P is normal based on the detected current value.

Furthermore, according to the neutron capture therapy apparatus 1B, it is possible to detect current values of a charged particle beam P using a plurality of current detection portions 61. Accordingly, it is possible to figure out which current detection portion 61 comes into contact with a charged particle beam P in an abnormal state based on the current value detected by each of the current detection portions 61. Accordingly, it is possible to obtain information on which position of the current detection portions 61 is irradiated with the charged particle beam P in the abnormal state.

Hereinabove, preferred embodiments of the present embodiments have been described. However, the present invention is not limited to the above-described embodiments, and may be modified within the scope not departing from the gist described in each claim, or may be applied to others.

In the above-described embodiments, the control when a position at which a target T is irradiated with a charged particle beam P is in an abnormal state is performed by the control portion 102, but the present invention is not limited thereto. For example, operators of the neutron capture therapy apparatuses 1A and 1B who observe the current values detected by the current detection portions 60 and 61 based on the waveform of the oscilloscope may stop irradiation of a target with a charged particle beam P while simultaneously observing the current value in the abnormal state.

The neutron capture therapy apparatuses 1A and 1B have been described in the above-described embodiments, but a radioisotope production apparatus or a nuclear transformation apparatus using a nuclear spallation reaction may be used instead of the neutron capture therapy apparatuses. In this case, similarly to the neutron capture therapy apparatuses 1A and 1B, the nuclear transformation apparatus includes an accelerator 10 which emits a charged particle beam P; a target T which is irradiated with the charged particle beam P to generate various nuclei or neutrons through a nuclear spallation reaction; a beam transport path 48 that transports the charged particle beam P which is emitted from the accelerator 10; and a current detection portion 60 which detects a current value of the charged particle beam P. The current detection portion 60 is provided inside the beam transport path 48 along an inner wall 48a in a state of being insulated from the inner wall 48a.

## Claims

1. A neutron capture therapy apparatus (1A, 1B) comprising:
an accelerator (10) which is configured to emit a charged particle beam (P);
a target (T) which is configured to be irradiated with the charged particle beam (P) to generate a neutron beam (N);
a beam transport path (48) that is configured to transport the charged particle beam (P), which is emitted from the accelerator (10), to the target (T), wherein the target (T) is disposed in a downstream end of the beam transport path (48) of the charged particle beam (P);
a current detection portion (60) which is configured to detect a current value of the charged particle beam (P), wherein the current detection portion (60) is provided inside the beam transport path (48) along an inner wall (48a) of the beam transport path (48) in a state of being insulated from the inner wall (48a), wherein the current detection portion (60) is insulated from the downstream end of the beam transport path (48) in which the target (T) is disposed, and wherein the current detection portion (60) is adapted to measure a current of the charged particle beam (P) which comes into contact with the current detection portion (60); and
a control portion (102) that is configured to stop the irradiation of the target (T) with the charged particle beam (P) by assuming that the position at which the target (T) is irradiated with the charged particle beam (P) is abnormal when the current value detected by the current detection portion (60) becomes less than or equal to a lower limit value which is smaller than a reference current value, or greater than or equal to an upper limit value which is greater than the reference current value, by setting a current value detected by the current detection portion (60) when the position at which the target (T) is irradiated with the charged particle beam (P) is normal, as the reference current value.

2. The neutron capture therapy apparatus (1A, 1B) according to claim 1,
wherein the current detection portion (60) is formed in an annular shape.

3. The neutron capture therapy apparatus (1A, 1B) according to claim 1,
wherein a plurality of the current detection portions (60) are provided along the inner wall (48a).

4. The neutron capture therapy apparatus (1A, 1B) according to any one of claims 1 to 3,
wherein the current detection portion (60) is provided so as to protrude from the inner wall (48a) side of the beam transport path (48) to the inside of the beam transport path (48).

5. The neutron capture therapy apparatus (1A, 1B) according to any one of claims 1 to 3, further comprising:
a control portion (102) which is configured to control the emission of the charged particle beam (P) based on the size of the current value detected by the current detection portion (60).

6. A nuclear transformation apparatus comprising:
an accelerator (10) which is configured to emit a charged particle beam (P) ;
a target (T) which is configured to be irradiated with the charged particle beam (P) to generate a predetermined nucleus or neutron through a nuclear spallation reaction;
a beam transport path (48) that is configured to transport the charged particle beam (P), which is emitted from the accelerator (10), to the target (T), wherein the target (T) is disposed in a downstream end of the beam transport path (48) of the charged particle beam (P);
a current detection portion (60) which is configured to detect a current value of the charged particle beam (P), wherein the current detection portion (60) is provided inside the beam transport path (48) along an inner wall (48a) of the beam transport path (48) in a state of being insulated from the inner wall (48a), wherein the current detection portion (60) is insulated from the downstream end of the beam transport path (48) in which the target (T) is disposed, and wherein the current detection portion (60) is adapted to measure a current of the charged particle beam (P) which comes into contact with the current detection portion (60);
a control portion (102) that is configured to control the irradiation of the target (T) with the charged particle beam (P) based on the size of the current value detected by the current detection portion (60) by assuming that the position at which the target (T) is irradiated with the charged particle beam (P) is abnormal when the current value detected by the current detection portion (60) becomes less than or equal to a lower limit value which is smaller than a reference current value, or greater than or equal to an upper limit value which is greater than the reference current value, by setting a current value detected by the current detection portion (60) when the position at which the target (T) is irradiated with the charged particle beam (P) is normal, as the reference current value.

## Patentansprüche

1. Neutroneneinfangtherapievorrichtung (1A, 1B), die Folgendes aufweist:
eine Beschleunigungsvorrichtung (10), die konfiguriert ist, um ein Strahl (P) geladener Teilchen zu emittieren;
ein Target bzw. Ziel (T), welches konfiguriert ist, um mit dem Strahl (P) geladener Teilchen bestrahlt zu werden, um einen Neutronenstrahl (N) zu erzeugen;
einen Strahltransportpfad (48), der konfiguriert ist, um den Strahl (P) geladener Teilchen, der von der Beschleunigungsvorrichtung (10) zu dem Ziel (T) emittiert worden ist, zu emittieren, wobei das Ziel (T) an einem nachgelagerten Ende des Strahltransportpfads (48) des Strahls (P) geladener Teilchen angeordnet ist;
einen Stromdetektionsteil (60), der konfiguriert ist, um einen Stromwert des Strahls (P) geladener Teilchen zu detektieren, wobei der Stromdetektionsteil (60) innerhalb des Strahltransportpfads (48) entlang einer Innenwand (48a) des Strahltransportpfads (48) in einem Zustand vorgesehen ist, der von der Innenwand (48a) isoliert ist, wobei der Stromdetektionsteil (60) von dem nachgelagerten Ende des Strahltransportpfads (48) isoliert ist, in dem das Ziel (T) angeordnet ist, und wobei der Stromdetektionsteil (60) ausgelegt ist, um einen Strom des Strahls (P) geladener Teilchen zu messen, der in Kontakt mit dem Stromdetektionsteil (60) kommt; und
einen Steuerteil (102), der konfiguriert ist, um die Bestrahlung des Ziels (T) mit dem Strahl (P) geladener Teile anzuhalten, und zwar indem angenommen wird, dass die Position, bei der das Ziel (T) mit dem Strahl (P) geladener Teilchen bestrahlt wird, abnormal ist, wenn der Stromwert, der durch den Stromdetektionsteil (60) detektiert wird, kleiner oder gleich einem unteren Grenzwert wird, der kleiner als ein Referenzstromwert ist, oder größer oder gleich einem oberen Grenzwert ist, der größer als der Referenzstromwert ist, indem ein Stromwert, der durch den Stromdetektionsteil (60) detektiert wird, wenn die Position, bei der das Ziel (T) mit dem Strahl (P) geladener Teilchen bestrahlt wird, normal ist, als der Referenzstromwert eingestellt wird.

2. Neutroneneinfangtherapievorrichtung (1A, 1B) gemäß Anspruch 1, wobei der Stromdetektionsteil (60) in einer ringförmigen Form gebildet ist.

3. Neutroneneinfangtherapievorrichtung (1A, 1B) gemäß Anspruch 1, wobei eine Vielzahl der Stromdetektionsteile (60) entlang der Innenwand (48a) vorgesehen ist.

4. Neutroneneinfangtherapievorrichtung (1A, 1B) gemäß einem der Ansprüche 1 bis 3, wobei der Stromdetektionsteil (60) so vorgesehen ist, dass er von der Seite der Innenwand (48a) des Strahltransportpfads (48) zu der Innenseite des Strahltransportpfads (48) vorragt.

5. Neutroneneinfangtherapievorrichtung (1A, 1B) gemäß einem der Ansprüche 1 bis 3, die ferner Folgendes aufweist:
einen Steuerteil (102), der konfiguriert ist, um die Emission des Strahls (P) geladener Teilchen basierend auf der Größe des Stromwerts zu steuern, der durch den Stromdetektionsteil (60) detektiert wird.

6. Nukleartransformationsvorrichtung, die Folgendes aufweist:
eine Beschleunigungsvorrichtung (10), die konfiguriert ist, um ein Strahl (P) geladener Teilchen zu emittieren;
ein Target bzw. Ziel (T), welches konfiguriert ist, um mit dem Strahl (P) geladener Teilchen bestrahlt zu werden, um einen vorbestimmten Kern oder ein Neutron durch eine Kernzertrümmerungsreaktion zu erzeugen;
einen Strahltransportpfad (48), der konfiguriert ist, um den Strahl (P) geladener Teilchen, der von der Beschleunigungsvorrichtung (10) emittiert wird, zu dem Ziel (T) zu emittieren, wobei das Ziel (T) an einem nachgelagerten Ende des Strahltransportpfads (48) des Strahls (P) geladener Teilchen angeordnet ist;
einen Stromdetektionsteil (60), der konfiguriert ist, um einen Stromwert des Strahls (P) geladener Teilchen zu detektieren, wobei der Stromdetektionsteil (60) innerhalb des Strahltransportpfads (48) entlang einer Innenwand (48a) des Strahltransportpfads (48) in einem Zustand vorgesehen ist, der von der Innenwand (48a) isoliert ist, wobei der Stromdetektionsteil (60) von dem nachgelagerten Ende des Strahltransportpfads (48) isoliert ist, in dem das Ziel (T) angeordnet ist, und wobei der Stromdetektionsteil (60) ausgelegt ist, um einen Strom des Strahls (P) geladener Teilchen zu messen, der in Kontakt mit dem Stromdetektionsteil (60) kommt;
einen Steuerteil (102), der konfiguriert ist, um die Bestrahlung des Ziels (T) mit dem Strahl (P) geladener Teile basierend auf der Größe des Stromwerts zu steuern, der durch den Stromdetektionsteil (60) detektiert wird, und zwar indem angenommen wird, dass die Position, bei der das Ziel (T) mit dem Strahl (P) geladener Teilchen bestrahlt wird, abnormal ist, wenn der Stromwert, der durch den Stromdetektionsteil (60) detektiert wird, kleiner oder gleich einem unteren Grenzwert ist, der kleiner als ein Referenzstromwert ist, oder größer oder gleich einem oberen Grenzwert ist, der größer als der Referenzstromwert ist, indem ein Stromwert, der durch den Stromdetektionsteil (60) detektiert wird, wenn die Position, bei der das Ziel (T) mit dem Strahl (P) geladener Teilchen bestrahlt wird, normal ist, als der Referenzstromwert eingestellt wird.

## Revendications

1. Appareil de thérapie par capture de neutrons (1A, 1B), comprenant :
un accélérateur (10) qui est agencé pour émettre un faisceau de particules chargées (P) ;
une cible (T) qui est agencée pour être irradiée par le faisceau de particules chargées (P) pour générer un faisceau de neutrons (N) ;
un chemin de transport de faisceau (48) qui est agencé pour transporter le faisceau de particules chargées (P), qui est émis à partir de l'accélérateur (10), vers la cible (T), la cible (T) étant disposée dans une extrémité aval du chemin de transport de faisceau (48) du faisceau de particules chargées (P) ;
une portion de détection de courant (60) qui est agencée pour détecter une valeur de courant du faisceau de particules chargées (P), la portion de détection de courant (60) étant prévue à l'intérieur du chemin de transport de faisceau (48) le long d'une paroi intérieure (48a) du chemin de transport de faisceau (48) dans un état où elle est isolée de la paroi intérieure (48a), la portion de détection de courant (60) étant isolée de l'extrémité aval du chemin de transport de faisceau (48) dans lequel la cible (T) est disposée, et la portion de détection de courant (60) étant adaptée à mesurer un courant du faisceau de particules chargées (P) qui vient en contact avec la portion de détection de courant (60) ; et
une portion de commande (102) qui est agencée pour stopper l'irradiation de la cible (T) par le faisceau de particules chargées (P) en supposant que la position à laquelle la cible (T) est irradiée par le faisceau de particules chargées (P) est anormale lorsque la valeur de courant détectée par la portion de détection de courant (60) devient inférieure ou égale à une valeur de limite inférieure qui est plus petite qu'une valeur de courant de référence, ou supérieure ou égale à une valeur de limite supérieure qui est plus grande que la valeur de courant de référence, en réglant une valeur de courant détectée par la portion de détection de courant (60) lorsque la position à laquelle la cible (T) est irradiée par le faisceau de particules chargées (P) est normale, comme valeur de courant de référence.

2. Appareil de thérapie par capture de neutrons (1A, 1B) selon la revendication 1,
dans lequel la portion de détection de courant (60) a une forme annulaire.

3. Appareil de thérapie par capture de neutrons (1A, 1B) selon la revendication 1,
dans lequel une pluralité des portions de détection de courant (60) est prévue le long de la paroi intérieure (48a).

4. Appareil de thérapie par capture de neutrons (1A, 1B) selon l'une quelconque des revendications 1 à 3,
dans lequel la portion de détection de courant (60) est prévue de manière à être saillante par rapport au côté de la paroi intérieure (48a) du chemin de transport de faisceau (48) vers l'intérieur du chemin de transport de faisceau (48).

5. Appareil de thérapie par capture de neutrons (1A, 1B) selon l'une quelconque des revendications 1 à 3, comprenant en outre :
une portion de commande (102) qui est agencée pour contrôler l'émission du faisceau de particules chargées (P) sur la base de la grandeur de la valeur de courant détectée par la portion de détection de courant (60).

6. Appareil de transformation nucléaire, comprenant :
un accélérateur (10) qui est agencé pour émettre un faisceau de particules chargées (P) ;
une cible (T) qui est agencée pour être irradiée par le faisceau de particules chargées (P) pour générer un noyau ou un neutron prédéterminé par l'intermédiaire d'une réaction de spallation nucléaire ;
un chemin de transport de faisceau (48) qui est agencé pour transporter le flux de particules chargées (P), qui est émis à partir de l'accélérateur (10), vers la cible (T), la cible (T) étant disposée dans une extrémité aval du chemin de transport de faisceau (48) du faisceau de particules chargées (P) ;
une portion de détection de courant (60) qui est agencée pour détecter une valeur de courant du faisceau de particules chargées (P), la portion de détection de courant (60) étant prévue à l'intérieur du chemin de transport de faisceau (48) le long d'une paroi intérieure (48a) du chemin de transport de faisceau (48) dans un état où elle est isolée de la paroi intérieure (48a), la portion de détection de courant (60) étant isolée de l'extrémité aval du chemin de transport de faisceau (48) dans lequel la cible (T) est disposée, et la portion de détection de courant (60) étant adaptée à mesurer un courant du faisceau de particules chargées (P) qui vient en contact avec la portion de détection de courant (60) ;
une portion de commande (102) qui est agencée pour contrôler l'irradiation de la cible (T) par le faisceau de particules chargées (P) sur la base de la grandeur de la valeur de courant détectée par la portion de détection de courant (60) en supposant que la position à laquelle la cible (T) est irradiée par le faisceau de particules chargées (P) est anormale lorsque la valeur de courant détectée par la portion de détection de courant (60) devient inférieure ou égale à une valeur de limite inférieure qui est plus petite qu'une valeur de courant de référence, ou supérieure ou égale à une valeur de limite supérieure qui est plus grande que la valeur de courant de référence, en réglant une valeur de courant détectée par la portion de détection de courant (60) lorsque la position à laquelle la cible (T) est irradiée par le faisceau de particules chargées (P) est normale, comme valeur de courant de référence.
